# EUROPEAN PATENT SPECIFICATION

(11) **EP 1 040 089 B1**
(45) Date of publication and mention of the grant of the patent: **02.10.2002**
(21) Application number: 98964729.2
(22) Date of filing: 15.12.1998
(51) Int. Cl.: C07C 5/22, C07C 4/12

(54) **META-XYLENE PRODUCTION PROCESS**
HERSTELLUNGSVERFAHREN FÜR META-XYLOL
PROCEDE DE PRODUCTION DE META-XYLENE

(30) Priority: 19.12.1997 US 995270
(43) Date of publication of application: 04.10.2000
(73) Proprietor: EXXONMOBIL OIL CORPORATION, Fairfax, VA 22037 (US)
(72) Inventor: HELLRING, Stuart, Damon, Pittsburgh, PA 15243 (US); STERN, David, Lawrence, Mount Laurel, NJ 08054 (US)
(74) Representative: Dew, Melvyn John
(86) International application number: PCT/US98/26587
(87) International publication number: WO 99/032421

(56) References cited:
- US-A- 4 899 011

## Description

This invention is directed to a process for producing *meta*-xylene and more specifically to a process for the catalytic conversion of ethylbenzene with minimized xylene isomerization, and subsequent purification to yield high-purity *meta*-xylene.

*Meta*-xylene is a valuable chemical intermediate useful in the production of specialty polyester resins for high-performance plastics, such as those used in blow-molded products. These materials find use in various products, including plastic bottles, and this market is expected to grow substantially in the coming years.

*Meta*-xylene may be derived from mixtures of C₈ aromatics separated from such raw materials as petroleum naphthas, particularly reformates, usually by selective solvent extraction. The C₈ aromatics in such mixtures and their properties are:

| | Freezing Point (°C) | Boiling Point (°C) | Density (Kg/m³) |
|---|---|---|---|
| Ethylbenzene | 95.0 | 136.2 | 869.9 |
| *Para*-xylene | 13.2 | 138.5 | 863.9 |
| *Meta*-xylene | 47.4 | 138.8 | 866.3 |
| *Ortho*-xylene | 25.4 | 144.0 | 883.1 |

Principal sources of the mixtures of C₈ aromatics are catalytically reformed naphthas and pyrolysis distillates. The C₈ aromatic fractions from these sources vary quite widely in composition but will usually be in the range of 10 to 32 wt.% ethylbenzene (EB) with the balance, xylenes, being divided approximately 50 wt.% *meta*-xylene and 25 wt.% each of *para*-xylene and *ortho*-xylene.

Individual isomer products may be separated from the naturally occurring mixtures by appropriate physical methods. Ethylbenzene may be separated by fractional distillation, although this is a costly operation. *Ortho*-xylene may be separated by fractional distillation, and it is so produced commercially. *Para*-xylene may be separated from the mixed isomers by fractional crystallization, selective adsorption (e.g., the Parex process), or membrane separation.

As is evident in the table of properties above, the boiling point of ethylbenzene is very close to those of *para*-xylene and *meta*-xylene, making separation of these components by distillation impractical. Ethylbenzene is therefore typically converted catalytically to benzene and/or additional xylenes during xylene isomerization to increase the yield of valuable xylene isomers. However, in most xylene isomerization processes, conversion of ethylbenzene is constrained by the need to hold conversion of xylenes to other compounds to acceptable levels. Thus, although catalytic removal of ethylbenzene is possible, operating conditions are generally selected to balance the disadvantages of xylene loss by transalkylation with the conversion of ethylbenzene.

There is currently one commercially practiced method for separation of *meta*-xylene, i.e., the Mitsubishi Gas Chemical Company (MGCC) process. See *Kirk-Othmer Encyclopedia of Chemical Technology,* 3rd ed., Vol. 24, p. 727 (1984), and the publications cited therein. This separations process requires treating a mixture of isomerized xylenes with HF-BF₃. Two layers are formed, i.e., a 1:1 molecular complex, *meta*-xylene-HBF₃ layer, and an organic layer containing the remaining xylenes. *Meta*-xylene is then recovered in 99% purity by thermal decomposition of the *meta*-xylene-HBF₃ complex. Although this method is used commercially by MGCC, the difficulties in dealing with HF-BF₃ make this process costly.

Thus, the state of the art of *meta*-xylene production is such that demand for this material continues to increase, while methods for preparing the material lag in commercial practicability. Presently, the processes for producing *meta*-xylene are limited by cost and purity considerations. It would, therefore, be highly desirable to provide a commercially acceptable process for the production of *meta*-xylene in substantially pure form.

According to the invention, there is provided a process for producing *meta*-xylene, comprising the steps of:
(a) converting a hydrocarbon feedstream comprising at least 5 wt.% ethylbenzene, at least 20 wt.% *meta*-xylene, and less than 5 wt.% *para*-xylene over a molecular sieve catalyst under ethylbenzene conversion conditions sufficient to provide a primary product stream depleted of more than 50%, preferably more than 95%, of the ethylbenzene present in the feedstream;
(b) stripping benzene and/or toluene by-products from the primary product stream to provide a secondary product stream comprising at least 75 wt.% mixed *ortho*-xylene and *meta*-xylene; and
(c) splitting the secondary product stream by removing substantially all of the *ortho*-xylene present therein to provide a tertiary product stream comprising at least 85 wt.%, and preferably at least 95 wt.%, *meta*-xylene.

Preferably, the process further comprises the step of distilling the tertiary product stream to obtain a distillate comprising at least 98 wt.% *meta*-xylene.

Preferably, the process further comprises the preliminary step of separating *para*-xylene from a mixed C₈ hydrocarbon feed comprising *para*-xylene, *ortho*-xylene, *meta*-xylene, and ethylbenzene to provide said hydrocarbon feedstream.

The process of the invention is capable of yielding high-purity *meta*-xylene in a straightforward and inexpensive process which can be incorporated into an existing xylene isomerization complex with minimal capital outlay but with high-purity *meta*-xylene productivity.

The invention will now be more particularly described with reference to the accompanying drawings in which:
Figure 1 is schematic illustration of a prior art xylene isomerization processing operation.
Figure 2 is a schematic illustration of a *meta*-xylene production process according to one embodiment of the invention.

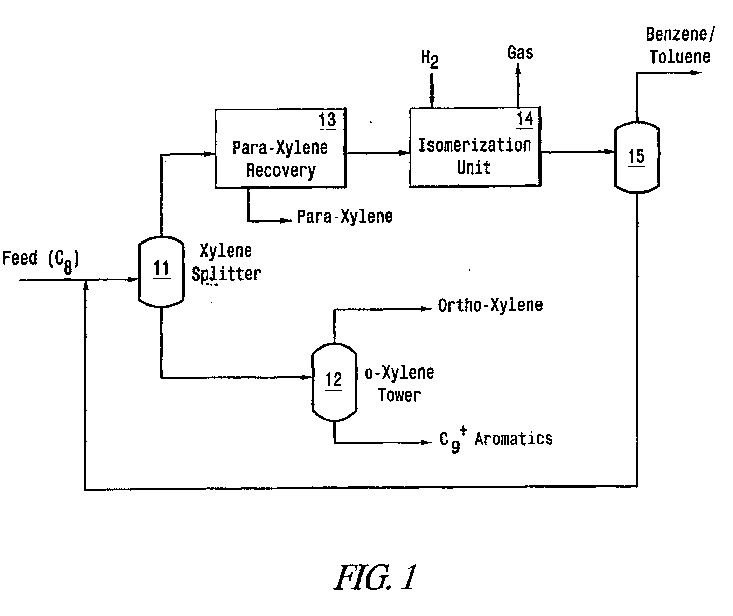

Referring to Figure 1, in the conventional xylene isomerization loop shown, a C₈ aromatics stream, such as a heart cut consisting essentially of C₈ aromatics, is fed through a xylene splitter 11 to remove *ortho*-xylene (and heavier C₉⁺ aromatics) from the mixed xylenes and ethylbenzene. The *ortho*-xylene and C₉⁺ aromatics are then fed to an *ortho*-xylene tower 12 where *ortho*-xylene is separated from the heavier components. The product from the xylene splitter 11 is fed to a *para*-xylene recovery unit 13 where a substantial proportion of the *para*-xylene is removed and recovered. The remaining other C₈ materials are then fed to a xylenes isomerization unit 14 together with a hydrogen cofeed. The isomerization product is fed to a distillation unit 15 to remove benzene and toluene byproducts, and the xylenes (with an increased proportion of *para*-xylene) is recycled to the xylene splitter 11.

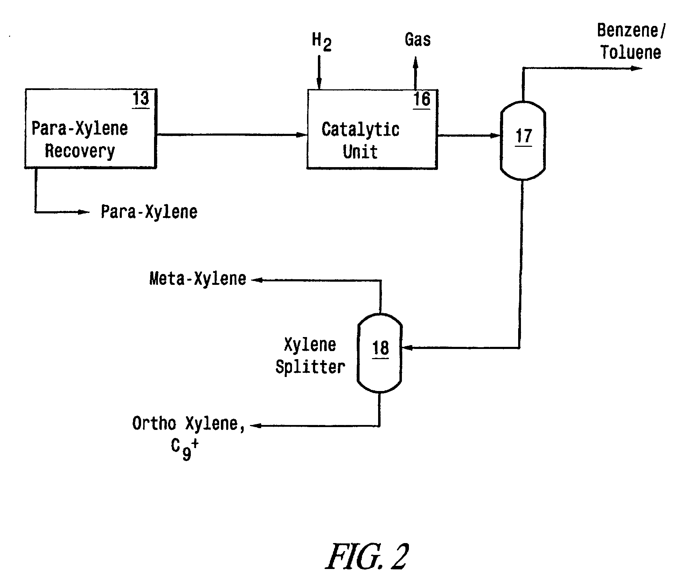

Referring to Figure 2, In the process of said one embodiment of the invention, a portion of the *para*-xylene effluent from the *para*-xylene recovery unit 13 of the xylene isomerization loop shown in Figure 1, is fed to a catalytic conversion unit 16 where a diffusionally restricted catalyst selectively converts the ethylbenzene and the small amount of *para*-xylene in the effluent at very high conversion levels. The resulting primary product stream is depleted of greater than 50%, preferably greater than 95%, and more preferably greater than 99%, of the ethylbenzene present in the feedstream. Optionally, benzene may be cofed to the conversion unit 16 to increase the conversion of *para*-xylene to toluene and thereby increase the purity of the *meta*-xylene product thus afforded.

The ethylbenzene-depleted primary product stream exiting the unit 16 is fed directly to benzene/toluene splitter 17, where the lighter weight by-products (benzene, toluene and light gas) are removed, preferably by distillation. Typically, this stripping process will yield a secondary product stream which contains at least 75 wt.%, preferably at least 85 wt.%, and more preferably at least 95 wt.%, mixed *ortho*-xylene and *meta*-xylene. Thereafter, the secondary product stream is fed to a xylene splitter 18 to remove the *ortho*-xylene and C₉⁺ aromatics. The light fraction from the splitter 18 is composed of very high purity *meta*-xylene. Typically this tertiary product stream comprises at least 85 wt.%, preferably at least 95 wt.%, and more preferably at least 98 wt.% *meta*-xylene. If desired, the tertiary product stream is distilled to further increase the purity of the *meta*-xylene.

It will be appreciated that in conventional xylene isomerization processes such as that shown in Figure 1, the xylene isomerization unit 11 may include an initial bed or reactor to effect conversion of ethylbenzene in the C₈ aromatic feed before the feed undergoes xylene isomerization. In the process of the invention, however, the ethylbenzene-depleted product from the catalytic conversion unit 16 passes directly to the benzene/toluene splitter 17 without undergoing an intermediate xylene isomerization step.

### Feedstock

In general, the feedstock to the process of the invention is aromatic C₈ mixture having an ethylbenzene content of 5 to 60 wt.%, an *ortho*-xylene content of 0 to 35 wt.%, a *meta*-xylene content 20 to 95 wt.% and a *para*-xylene content in the approximate range of 0 to 5 wt.%. Preferably, the feedstock will contain at least 30 wt.%, more preferably at least 40 wt.%, *meta*-xylene. Feedstocks meeting this standard are termed "*meta*-xylene-rich" feedstocks. A preferred feedstock is obtained as the effluent stream from a *para*-xylene recovery unit.

In the process of the invention the ethylbenzene in the feedstock is selectively converted, largely by dealkylation to produce benzene and light gas, while isomerization of the *meta*-xylene and any *ortho*-xylene is minimized so that the primary product stream exiting the catalytic unit 16 Is substantially free of *meta*-xylene co-boilers.

The C₈ feedstream may also comprise added benzene as a cofeed to facilitate *para*-xylene conversion by transalkylation to produce toluene. Moreover, in addition to the above, the aromatic C₈ mixture may contain non-aromatic hydrocarbons, i.e., naphthenes and paraffins in an amount up to 30 wt.%. In a preferred embodiment, the invention provides means to process a mixture of C₈ aromatics such as that derived after other known processing steps such as solvent extraction and distillation from catalytic reforming of a petroleum naphtha to a mixture of reduced ethylbenzene and *para*-xylene content.

### Process Conditions

In accordance with the present invention, the above described feedstock is contacted with the catalyst system under suitable conversion conditions to effect ethylbenzene conversion and optionally *para*-xylene conversion. Suitable conversion conditions include a temperature of 200° to 550°C, preferably 325° to 475°C, a pressure of 0 to 1000 psig (100 to 7000 kPa), preferably 50 to 400 psig (450 to 2850 kPa), a WHSV of 0.1 to 200 hr⁻¹, preferably 3 to 50 hr⁻¹, and an H₂/HC molar ratio of 0.2 to 10, preferably 1 to 5.

### Catalyst System

The principal function of the catalyst system in the process of the invention is to effect a high degree of conversion of the ethylbenzene in the feed with minimal isomerization of the xylenes, particularly the *meta*-xylene and any *ortho*-xylene which also may be present, so that there is no net p-xylene make. This is preferably achieved by use of a molecular sieve catalyst which has limited diffusion for xylenes, particularly the larger isomers, *meta*-xylene and *ortho*-xylene. In this way the accessibility of the internal acid sites of the catalyst to the *meta*-xylene and *ortho*-xylene is limited. In particular, it has been found that the catalyst preferably has an *ortho*-xylene sorption time (t_{0 3}), i.e., the time required to achieve 30% of its equilibrium *ortho*-xylene sorption capacity, of greater than 50 minutes when measured at 120°C and an *ortho*-xylene pressure of 4.5 ± 0.8 mm of mercury (493 Pa to 707 Pa). Such sorption measurements may be carried out gravimetrically in a thermal balance.

The catalyst used in the process of the invention is also preferably selected so as to have extremely low xylene isomerization activity, especially at its external acid sites. For example, the xylene isomerization activity of the catalyst is preferably such that the catalyst produces less than 12 wt.% *para*-xylene when contacting a feed containing 60 wt.% *meta*-xylene, 20 wt.% *ortho*-xylene, and 20 wt.% ethylbenzene at a temperature of 426.7°C, a pressure of 150 psig (1136 kPaa), a weight hourly space velocity (WHSV) of 20 hr⁻¹, and a hydrogen to hydrocarbon (H₂/HC) molar ratio of 1.

Catalysts useful in this invention comprise catalytic molecular sieves, such as zeolites and preferably intermediate pore size zeolites, that is zeolites having a pore size of 5Å to 7 Å. Preferably, the molecular sieve has a Constraint Index of 1 to 12 (as described in U.S. Patent No. 4,016,218).

Examples of intermediate pore size zeolites useful in this invention include ZSM-5 (U.S. Patent No. 3.702,886 and Re. 29,948); ZSM-11 (U.S. Patent No. 3,709,979); ZSM-12 (U.S. Patent No. 3,832,449); ZSM-22 (U.S. Patent No. 4,556,447); ZSM-23 (U.S. Patent No. 4,076,842); ZSM-35 (U.S. Patent No. 4,016,245); ZSM-57 (U.S. Patent No. 4,046,685); and ZSM-58 (U.S. Patent No. 4,417,780).

Other useful catalytic molecular sieves include MCM-22, MCM-36, MCM-49, MCM-56, silicoaluminophosphates such as SAPO-5, SAPO-11, and other zeolites including zeolite X, zeolite Y, and zeolite Beta.

As synthesized, the molecular sieves listed above may not exhibit the desired xylene diffusion characteristics and xylene isomerization activity required to effect ethylbenzene conversion with minimal xylene isomerization. However, the requisite diffusional properties may be provided by controlling the crystal size of the sieve and/or by coating the catalyst with a selectivating agent which inhibits the diffusivity of the molecular sieve, particularly, the diffusivity of the molecular sieve to *ortho*-xylene and *meta*-xylene. Preferably, the selectivating agent is silica and is produced by applying to the catalyst one or more coatings of an organosilicon selectivating agent in a liquid carrier followed, after each coating, by calcining the catalyst in an oxygen-containing atmosphere. Such a silicon selectivation process is described in detail in International Publication No. WO 96/16005. Alternatively, the desired diffusional properties may be achieved through the use of coke selectivation, as described in U.S. Patent No. 4,097,543, either alone or in combination with silicon selectivation. Selectivation with silica and/or coke reduces the external acid activity and hence the xylene isomerization activity of the catalyst.

The suitable molecular sieve may be employed in combination with a support or binder material such as, for example, a porous inorganic oxide support or a clay binder. For example, it may be desirable to formulate the catalyst of the invention with another material resistant to the temperature and other conditions of the hydrocarbon conversion process. Illustrative examples of binder materials include synthetic or naturally occurring substances as well as inorganic materials such as clay, silica, and/or metal oxides, such as alumina, vanadia, beryllia, thoria, magnesia, titania, zirconia, boria, and combinations thereof. The preferred binder is primarily silica. The metal oxides may be naturally occurring or in the form of gelatinous precipitates or gels including mixtures of silica and metal oxides.

Naturally occurring clays that can be composited with the molecular sieve include those of the montmorillonite and kaolin families, which families include the subbentonites and the kaolins commonly known as Dixie, McNamee, Georgia, and Florida clays, or others in which the main mineral constituent is halloysite, kaolinite, dickite, nacrite, or anauxite. Suitable clay materials include, by way of example, bentonite and kieselguhr. Such clays can be used in the raw state as originally mined or initially subjected to calcination, acid treatment, or chemical modification.

The relative proportion of suitable crystalline molecular sieve to the total composition of catalyst and binder or support may be from 1 to 99 wt.%, preferably from 30 to 90 wt.%, and more preferably from 50 to 80 wt.%, of the composition.

In order for the catalyst used in the process of the invention to be effective to convert ethylbenzene with minimal xylene isomerization, it is preferable that the catalyst has an alpha value of at least 5, more preferably from 75 to 5000 and typically from 100 to 2000. The alpha value is an approximate indication of the catalytic cracking activity of the catalyst compared to a standard catalyst and it gives the relative rate constant (rate of normal hexane conversion per volume of catalyst per unit time). It is based on the activity of an amorphous silica-alumina cracking catalyst taken as an alpha of 1 (Rate Constant = 0.016 sec¹). The Alpha Test is described in U.S. Patent No. 3,354,078 and in *J. Catalysis* 4:522-529 (August 1965): *J. Catalysis* 6:278 (1966); and *J. Catalysis* 61:395 (1980).

A hydrogenation-dehydrogenation functional metal can be incorporated into the catalyst of the invention. Such metals are known in the art to reduce ethylbenzene by-product in hydrocarbon conversion processes, see, e.g., U.S. Patent No. 5,498,814.

Any metal possessing the desired hydrogenation-dehydrogenation function can be used in the modification method of the invention. These are termed "functional metals". Examples of such functional metals include the oxide, hydroxide, sulfide, or free metal (i.e., zero valent) forms of metals in the Groups 3 to 15 of the periodic table. Preferred metals include Group 8, 9, and 10 metals (i.e., Pt, Pd, Ir, Rh, Os, Ru, Ni, Co, and Fe), Group 7 metals (i.e., Mn, Tc, and Re), Group 6 metals (i.e., Cr, Mo, and W), Group 15 metals (i.e., Sb and Bi), Group 14 metals (i.e., Sn and Pb), Group 13 metals (i.e., Ga and In), Group 11 metals (i.e., Cu, Ag, and Au), and Group 12 metals (i.e., Zn, Cd, and Hg). Noble metals (i.e., Pt, Pd, Ir, Rh, Os, Re, Ru, Mo, and W) are preferred.

Combinations or mixtures of catalytic forms of such noble or non-noble metal, such as combinations of Pt with Sn, may be used. The valence state of the metal is preferably reduced, e.g., when this component is in the form of an oxide or hydroxide. The reduced valence state of the functional metal may be attained, *in situ*, during the course of a reaction, when a reducing agent, such as hydrogen, is included in the feed to the reaction.

The functional metal may be incorporated into the catalyst by methods known in the art, such as ion exchange, impregnation, or physical admixture. For example, solutions of appropriate metal salts may be contacted with the remaining catalyst components, either before or after selectivation of the catalyst, under conditions sufficient to combine the respective components. The amount of functional metal incorporated in the catalyst can vary widely depending, for example, on the hydrogenation activity of the metal employed. Generally, however, the amount of the functional metal is suitably from 0.001 to 10 wt.%, preferably from 0.05 to 5 wt.%, more preferably from 0.1 to 2 wt.%, based on the total weight of the modified catalyst.

### EXAMPLE

An HZSM-5 catalyst preparation (65/35 zeolite/binder) was subjected to a four-fold silicone selectivation procedure. In each selectivation sequence, the catalyst was contacted with Dow-550 dissolved in decane and, after the decane was stripped, the catalyst was calcined at 540°C in nitrogen, and then in air.

Platinum was exchanged into the selectivated catalyst using a conventional exchange technique to produce a modified catalyst containing 0.1 wt.% Pt. Specifically, 0.0271 g of Pt(NH₃)₄Cl₂ H₂O was dissolved in 60 mL distilled, deionized water in a beaker with a stirbar. The beaker was equipped to support a Büchner funnel (a "ceramic thimble"). Catalyst (15 g) was loaded into a Büchner funnel, which was then placed in this solution. The pH of the solution then dropped to pH 3 and ammonium hydroxide (0.1 N) was added dropwise to maintain the pH at between 4 and 7. Following the exchange, the catalyst was dried and calcined at 350°C for 2 hr. The calcined catalyst was then crushed and sized to 14/20 mesh.

A microunit evaluation was conducted on the resultant catalyst in an automated unit with on-line gas chromatograph (GC) sampling. Catalyst (0.75 g) was loaded into a 3/8-inch diameter, stainless steel tube reactor (with sand as inert packing material). The reactor was pressurized to 150 psig (1135 Kpa) with N₂, and heated to 350°C under flowing nitrogen. The catalyst was then reduced by interrupting the N₂ flow, and introducing H₂ flow at a rate of 100 mUmin. After reducing for 2 hr, the reactor was heated to reaction temperature and feed was introduced. The feed was blended from *meta*-xylene, *ortho*-xylene, and ethylbenzene (Aldrich "HPLC Grade").

Catalytic evaluation was conducted at 10 hr⁻¹ WHSV, 1 H₂/HC, 350°C and 150 psig (1135 Kpa) pressure. The results are presented in Table 1, below.

**TABLE 1**

| Yields (wt.%) | Feed | Results |
|---|---|---|
| C₅ | - | 5.3 |
| Benzene | - | 14.3 |
| Toluene | - | 1.6 |
| Ethylbenzene | 20 | 0.15 |
| *Para*-Xylene | 0 | 0.7 |
| *Meta*-Xylene | 60 | 58.2 |
| *Ortho*-Xylene | 20 | 19.7 |
| C₉⁺ | - | 0.1 |
| Ethylbenzene Conversion (%) | - | 99.3 |
| Xylene Loss | - | 1.7 |

The results of this evaluation, as summarized above, demonstrate that extremely high ethylbenzene conversion can be achieved using the method of the invention. Although *para*-xylene was not present as a feed component, the amount of *para*-xylene formed was very small. *Para*-xylene can also be selectively converted to toluene and trimethylbenzene (via disproportionation) or to toluene with benzene (via reverse toluene disproportionation). Given these results, we calculated *meta*-xylene purity, defined as the amount of *meta*-xylene divided by the sum of the amounts of *meta*-xylene and its coboilers (i.e., ethylbenzene, *para*-xylene). In this case the *meta*-xylene purity was 98.6%.

These results clearly show that high-purity *meta*-xylene can be produced by catalytically converting ethylbenzene, coupled with distillation. Although this feed did not include *para*-xylene, its inclusion in the feed would not be expected to significantly change these results. Furthermore, benzene may be cofed to facilitate the conversion of *para*-xylene to compounds such as toluene and trimethylbenzene.

## Claims

1. A process for producing *meta*-xylene, comprising the steps of:
(a) converting a hydrocarbon feedstream comprising at least 5 wt.% ethylbenzene, at least 20 wt.% *meta*-xylene, and less than 5 wt.% *para*-xylene over a molecular sieve catalyst under ethylbenzene conversion conditions sufficient to provide a primary product stream depleted of more than 50% of the ethylbenzene present in the feedstream;
(b) stripping benzene and/or toluene by-products from the primary product stream to provide a secondary product stream comprising at least 75 wt.% mixed *ortho*-xylene and *meta*-xylene; and
(c) splitting the secondary product stream by removing substantially all of the *ortho*-xylene present therein to provide a tertiary product stream comprising at least 85 wt.% *meta*-xylene.

2. The process of Claim 1, further comprising the step of distilling the tertiary product stream to obtain a distillate having further increased *meta*-xylene content.

3. The process of Claim 1, further comprising cofeeding benzene with the hydrocarbon feedstream.

4. The process of Claim 1, wherein the ethylbenzene conversion conditions comprise a temperature of 200° to 550°C, a pressure of 0 to 1000 psig (100 to 7000 kPa), a WHSV of 0.1 to 200 hr⁻¹, and an H₂/HC molar ratio of 0.2 to 10.

5. The process of Claim 1, wherein the catalyst has an *ortho*-xylene t_{0.3} sorption time of greater than 50 min at 4.5 ± 0.8 mm Hg (493 Pa-707 Pa) and 120°C.

6. The process of Claim 1, wherein the catalyst produces less than 12 wt.% *para*-xylene when contacting a feed containing 60 wt.% *meta*-xylene, 20 wt.% *ortho*-xylene, and 20 wt.% ethylbenzene at a temperature of 426.7°C, a pressure of 150 psig (1135 Kpa) a WHSV of 20 hr⁻¹, and a H₂/HC molar ratio of 1.

7. The process of Claim 1, wherein the molecular sieve is selected from the group consisting of ZSM-5, ZSM-11, ZSM-12, ZSM-22, ZSM-23, ZSM-35, ZSM-57, ZSM-58, SAPO-5, SAPO-11, zeolite Beta, zeolite X, zeolite Y, MCM-22, MCM-36, MCM-49, and MCM-56.

8. The process of Claim 7, wherein the molecular sieve is ZSM-5.

9. The process of Claim 1, wherein the catalyst has been modified by silicon and/or coke selectivation.

10. The process of Claim 1, wherein the catalyst includes a hydrogenation-dehydrogenation functional metal.

## Patentansprüche

1. Verfahren zur Herstellung von meta-Xylol, umfassend die Stufen:
(a) Umwandeln eines Kohlenwasserstoffeinsatzmaterialstroms, der mindestens 5 Gew.% Ethylbenzol, mindestens 20 Gew.% meta-Xylol und weniger als 5 Gew.% para-Xylol umfasst, über einem Molekularsiebkatalysator unter Ethylbenzolumwandlungsbedingungen, die ausreichen, um einen Primärproduktstrom zu liefern, der an mehr als 50 % des in dem Einsatzmaterialstrom vorhandenen Ethylbenzol verarmt ist;
(b) Strippen von Benzol- und/oder Toluol-Nebenprodukten aus dem Primärproduktstrom, um einen Sekundärproduktstrom zu liefern, der mindestens 75 Gew.% gemischtes ortho-Xylol und meta-Xylol umfasst und
(c) Aufspalten des Sekundärproduktstroms, indem im Wesentlichen alles darin vorhandene ortho-Xylol entfernt wird, um einen Tertiärproduktstrom zu liefern, der mindestens 85 Gew.% meta-Xylol umfasst.

2. Verfahren nach Anspruch 1, das ferner die Stufe des Destillierens des Tertiärproduktstroms umfasst, um ein Destillat mit weiter erhöhtem meta-Xylol-Gehalt zu erhalten.

3. Verfahren nach Anspruch 1, das ferner das gemeinsame Einspeisen von Benzol mit dem Kohlenwasserstoffeinsatzmaterialstrom umfasst.

4. Verfahren nach Anspruch 1, bei dem die Ethylbenzolumwandlungsbedingungen eine Temperatur von 200° bis 550°C, einen Druck von 0 bis 1000 psig (100 bis 7000 kPa), ein WHSV von 0,1 bis 200 h⁻¹ und ein H₂/HC-Molverhältnis von 0,2 bis 10 umfassen.

5. Verfahren nach Anspruch 1, bei dem der Katalysator eine ortho-Xylol-t_{0,3}-Sorptionszeit von mehr als 50 Minuten bei 4,5 ± 0,8 mm Hg (493 Pa bis 707 Pa) und 120°C aufweist.

6. Verfahren nach Anspruch 1, bei dem der Katalysator weniger als 12 Gew.% para-Xylol erzeugt, wenn er ein Einsatzmaterial, das 60 Gew.% meta-Xylol, 20 Gew.% ortho-Xylol und 20 Gew.% Ethylbenzol enthält, bei einer Temperatur von 426,7°C, einem Druck von 150 psig (1135 KPa), einem WHSV von 20 h⁻¹ und einem H₂/HC-Molverhältnis von 1 kontaktiert.

7. Verfahren nach Anspruch 1, bei dem das Molekularsieb ausgewählt ist äus der Gruppe bestehend aus ZSM-5, ZSM-11, ZSM-12. ZSM-22, ZSM-23, ZSM-35, ZSM-57, ZSM-58, SAPO-5, SAPO-11, Zeolith-ß, Zeolith X, Zeolith Y, MCM-22, MCM-36, MCM-49 und MCM-56.

8. Verfahren nach Anspruch 7, bei dem das Molekularsieb ZSM-5 ist.

9. Verfahren nach Anspruch 1, bei dem der Katalysator durch Silicium- und/oder Koks-Selektivierung modifiziert worden ist.

10. Verfahren nach Anspruch 1, bei dem der Katalysator funktionelles Hydrier/Dehydrier-Metall einschließt.

## Revendications

1. Procédé pour la production de méta-xylène, comprenant les étapes consistant :
(a) à convertir un courant d'hydrocarbures d'alimentation comprenant au moins 5 % en poids d'éthylbenzène, au moins 20 % en poids de méta-xylène et moins de 5 % en poids de para-xylène sur un catalyseur à base de tamis moléculaire dans des conditions de conversion d'éthylbenzène suffisantes pour former un courant primaire de produits débarrassé de plus de 50 % de l'éthylbenzène présent dans le courant d'alimentation ;
(b) à entraîner les sous-produits consistant en benzène et/ou toluène du courant primaire de produits pour obtenir un courant secondaire de produits comprenant au moins 75 % en poids d'un mélange d'ortho-xylène et de méta-xylène ; et
(c) à scinder le courant secondaire de produits en séparant pratiquement la totalité de l'ortho-xylène présent dans ce courant pour former un courant tertiaire de produits comprenant au moins 85 % en poids de méta-xylène.

2. Procédé suivant la revendication 1, comprenant en outre l'étape de distillation du courant tertiaire de produits pour obtenir un distillat ayant une teneur en méta-xylène davantage accrue.

3. Procédé suivant la revendication 1, comprenant en outre l'introduction conjointe de benzène avec le courant d'hydrocarbures d'alimentation.

4. Procédé suivant la revendication 1, dans lequel les conditions de conversion d'éthylbenzène comprennent une température comprise dans l'intervalle de 200° à 550°C, une pression comprise dans l'intervalle de 0 à 1000 psig (100 à 7000 kPa), une VSHP de 0,1 à 200 h⁻¹ et un rapport molaire H₂/HC de 0,2 à 10.

5. Procédé suivant la revendication 1, dans lequel le catalyseur a un temps de sorption t_{0,3} de l'ortho-xylène supérieur à 50 min à une pression de 4,5 ± 0,8 mm de Hg (493 - 707 Pa) à une température de 120°C.

6. Procédé suivant la revendication 1, dans lequel le catalyseur produit moins de 12 % en poids de para-xylène lors de sa mise en contact avec une charge d'alimentation contenant 60 % en poids de méta-xylène, 20 % en poids d'ortho-xylène et 20 % en poids d'éthylbenzène à une température de 426,7°C, une pression de 150 psig (1135 kPa), une VSHP de 20 h⁻¹ et un rapport molaire H₂/HC égal à 1.

7. Procédé suivant la revendication 1, dans lequel le tamis moléculaire est choisi dans le groupe consistant en les ZSM-5, ZSM-11, ZSM-12, ZSM-22, ZSM-23, ZSM-35, ZSM-57, ZMS-58, SAPO-5, SAPO-11, la zéolite bêta, la zéolite X, la zéolite Y, les MCM-22, MCM-36, MCM-49 et MCM-56.

8. Procédé suivant la revendication 7, dans lequel le tamis moléculaire consiste en ZSM-5.

9. Procédé suivant la revendication 1, dans lequel le catalyseur a été modifié par activation sélective avec du silicium et/ou du coke.

10. Procédé suivant la revendication 1, dans lequel le catalyseur comprend un métal à fonction d'hydrogénation-déshydrogénation.
